# EUROPÄISCHE PATENTANMELDUNG

(11) **EP 3 136 102 A1**
(43) Veröffentlichungstag der Anmeldung: **01.03.2017**
(21) Anmeldenummer: 16001864.4
(22) Anmeldetag: 24.08.2016
(51) Int. Cl.: G01N 33/68, G01N 33/543

(54) **VERFAHREN ZUR BESTIMMUNG EINES ANTIKÖRPERBASIERTEN ANALYTEN IN KÖRPERFLÜSSIGKEITEN EINES SÄUGERS**

(30) Priorität: 26.08.2015 DE 102015011203
(71) Anmelder: Fraunhofer-Gesellschaft zur Förderung der angewandten Forschung e.V., 80686 München (DE)
(72) Erfinder: Grießner, Matthias, 03055 Cottbus (DE); Rümpel, Erik, 14473 Potsdam (DE); Ehrentreich-Förster, Eva, 14558 Potsdam/ Nuthetal (DE)
(74) Vertreter: v. Bezold & Partner Patentanwälte - PartG mbB

(57) **Zusammenfassung**

Die Erfindung betrifft ein Verfahren zur Bestimmung eines antikörperbasierten Analyten, insbesondere von Testosteron, in Körperflüssigkeiten eines Säugers und eine zur Durchführung des Verfahrens geeignete Vorrichtung.

Das erfindungsgemäße Verfahren umfasst mindestens die folgenden Schritte:
a) Kontaktieren einer Körperflüssigkeitsprobe des Säugers mit einem Anti-Analyt-Antikörper, wobei freier Analyt in der Probe an den Anti-Analyt-Antikörper gebunden wird und eine vollständige oder teilweise Absättigung der Analyt-Bindungsplätze des Antikörpers erfolgt,
b) Kontaktieren des nach Schritt a) erhaltenen Anti-Analyt-Antikörpers mit Referenz-Analyt und gegebenenfalls Bindung des Referenz-Analyten an noch vorhandene freie Bindungsplätze, wobei durch die Bindung ein detektierbares Signal erzeugt oder verändert wird,
c) Messen des erzeugten oder veränderten Signals, und
d) gegebenenfalls Vergleichen des gemessenen Signals mit Referenzwerten.

Vorzugsweise liegt der Referenz-Analyt dabei auf einer Oberfläche immobilisiert oder als multimeres Analyt-Konjugat vor.

## Beschreibung

### Hintergrund

Eine weitläufig durchgeführte Maßnahme in der Schweinehaltung für die Fleischproduktion ist die sogenannte "Ferkelkastration". Das Ziel ist es dabei die Bildung von Geschlechtshormonen, wie Testosteron zu verhindern, welches zum typischen "Ebergeruch" beiträgt und das Fleisch nur bedingt genießbar macht. Kastrierte Ferkel zeichnen sich weiterhin durch bessere Fleischqualität, schnelleren Fleischzuwachs und ein besseres Sozialverhalten aus. Die Kastration der Ferkel wird zumeist ohne Betäubung durchgeführt und wirkt damit traumatisierend für die betroffenen Tiere. Das Bundeslandwirtschaftsministerium hat aus diesem Grund ab dem 1. Januar 2017 ein Verbot der betäubungslosen Ferkelkastration verabschiedet.

Um weiterhin der Bildung des "Ebergeruchs" entgegenzuwirken, ergeben sich hauptsächlich drei Möglichkeiten. Dies sind die Kastration unter Narkose, die "Jungebermast" und die Impfung gegen Geschlechtshormone. Die Kastration unter Narkose zeigt sich nachteilhaft, da hierbei mit Narkosemitteln wie Isofluran umgegangen werden muss, die besondere Schutzmaßnahmen erfordern. Das Ferkel empfindet nach der Narkose ebenfalls noch Schmerzen und mitunter kann eine falsche Dosierung zum Tod führen. Die "Jungebermast" verzichtet auf Eingriffe und schlachtet die Tiere noch vor der Bildung des "Ebergeruchs". Allerdings kommt es bei dieser Methode nicht zur Verbesserung des Sozialverhaltens, wodurch Verletzungen durch Kämpfe entstehen können. Die Impfung bspw. mit Improvac® verhindert die Bildung der Geschlechtshormone und zeigt auch sonst alle Vorteil der Kastration. Mitunter kommt es dabei vor, dass das Fleisch der Tiere trotz Impfung noch "Ebergeruch" aufweist, was allerdings erst nach der Schlachtung realisiert werden kann. Somit ist dies nicht mehr zum Verzehr geeignet und das Tier umsonst getötet worden.

Von Interesse sind daher Nachweisverfahren für Testosteron, die eine optische Kontrolle der Impfung ermöglichen und damit das Risiko einer "Fehlschlachtung" verhindern. Ebenso kann die Produktion von Geschlechtshormonen bei der "Jungebermast" mit solchen Nachweisverfahren überprüft werden.

Bisherige Ansätze nutzen mitunter teure Verfahren, wie gaschromatographische Untersuchungen oder aber die Verwendung von Massenspektrometern, welche nicht für den "Feldeinsatz" geeignet sind. (Erfassung von "Ebergeruch" mit Spurenmesstechnik über die Gasphase, Boeker, Haas, Schulze-Lammers, Tholen; Züchtungskunde 84, (5) S. 439-458 2012 ISSN 0044-5401; Verlag Eugen Ulmer, Stuttgart).

EP 12333326 A1 offenbart ein Verfahren zum Nachweis von Ebergeruch durch Bestimmung der Skatol- und Androstenongehalte in einer biologischen Probe (Messungen aus der Gasphase, welche durch alle Gerüche beeinflusst ist.)

Die vorliegende Erfindung beruht auf dem Befund, dass diese Nachteile des Standes der Technik durch das erfindungsgemäße antikörper-basierte Verfahren zur Bestimmung des Testosterongehaltes in Körperflüssigkeiten eines Säugers vermieden oder minimiert werden können.

### Beschreibung der Erfindung

Das Verfahren zur Bestimmung eines spezifischen Analyten in Körperflüssigkeiten eines Säugers unter Verwendung eines

Anti-Analyt-Antikörpers nach Anspruch 1 umfasst die folgenden Schritte:
a) Kontaktieren einer Körperflüssigkeitsprobe des Säugers mit einem Anti-Analyt-Antikörper, wobei freier Analyt in der Probe an den Anti-Analyt-Antikörper gebunden wird und eine vollständige oder teilweise Absättigung der Analyt-Bindungsplätze des Antikörpers erfolgt,
b) Kontaktieren des nach Schritt a) erhaltenen Anti-Analyt-Antikörpers mit Referenz-Analyt und gegebenenfalls Bindung des Referenz-Analyten an noch vorhandene freie Bindungsplätze, wobei durch die Bindung ein detektierbares Signal erzeugt oder verändert wird,
c) Messen des erzeugten oder veränderten Signals, und
d) gegebenenfalls Vergleichen des gemessenen Signals mit Referenzwerten.

Der Säuger kann grundsätzlich jeder Säuger, insbesondere jeder männliche Säuger sein. Vorzugsweise ist der Säuger aus der Gruppe ausgewählt, welche Schweine und andere Nutztiere umfasst.

Als Analyte kommen grundsätzlich alle mit einem spezifischen Antikörper nachweisbare Proteine, Peptide, Hormone und niedermolekulare Verbindungen in Frage.

Vorzugsweise ist der Analyt aus der Gruppe der Hormone oder gesundheitsgefährdenden Stoffe ausgewählt.

Besonders bevorzugt ist als Analyt jedoch Testosteron.

In speziellen Ausführungsformen des erfindungsgemäßen Verfahrens liegt der Referenz-Analyt auf einer Oberfläche immobilisiert oder als multimeres Analyt-Konjugat vor.

Eine weitere spezielle Ausführungsform des erfindungsgemäßen Verfahrens ist dadurch gekennzeichnet, dass der Anti-Analyt-Antikörper in Schritt b) auch mit einem markierten sekundären Antikörper gegen den primären Anti-Analyt-Antikörper kontaktiert wird und die Bindung des markierten sekundären Antikörpers an das Konjugat von Anti-Analyt-Antikörper und Referenz-Analyt ein detektierbares Signal erzeugt oder verändert.

Das Signal kann grundsätzlich jede Art von detektierbarem Signal sein, welches eine Veränderung eines chemischen oder physikalischen Parameters anzeigt. Diese Veränderung kann z.B. die Bildung oder das Verschwinden eines bestimmten Produkts, z.B. eines farbigen oder fluoreszierenden Produkts, oder eine Veränderung im Reaktionsmilieu, z.B. eine pH-Änderung, Absorptionsänderung, Viskositätsänderung, eine Aggregatbildung, Immobilisierung etc. sein.

Das Signal kann beispielsweise ein optisches, elektrochemisches, (elektro)magnetisches oder radioaktives Signal sein. Vorzugsweise ist das Signal ein optisches Signal, das z.B. eine Farbänderung oder Fluoreszenzänderung anzeigt.

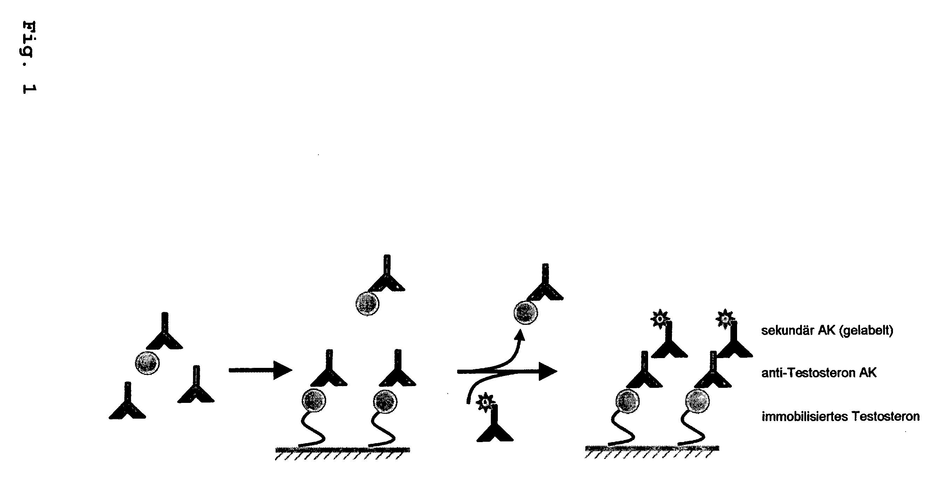

Eine bevorzugte Ausführungsform des erfindungsgemäßen Verfahrens ist ein kompetitiver Assay wie in Fig. 1 schematisch dargestellt.

Dabei wird ein anti-Testosteron-Antikörper mit der Probe inkubiert. Eine niedrige Testosteronkonzentration in der Probe führt dazu, dass eine geringe Anzahl an Paratop-Bindungsplätzen am anti-Testosteron-Antikörper belegt ist. Diese Antikörper stehen weiteren Bindungsereignissen zur Verfügung und können folglich in einem weiteren Schritt an oberflächenimmobilisiertes Testosteron binden. Mit einem sekundären Antikörper werden die Bindungsereignisse über die Auslesung eines Signals, beispielsweise eines Fluoreszenzsignals, nachgewiesen. Das Verfahren ermöglicht neben der Bestimmung von Testosteron ebenfalls die Bestimmung von weiteren Analyten wie z.B. Krankheitserregern.

Eine speziellere Ausführungsform des Verfahrens ist dadurch gekennzeichnet, dass der Referenz-Analyt auf einer Oberfläche immobilisiert ist und der Nachweis der Bindung des Anti-Analyt-Antikörpers an die Oberfläche mit einem markierten sekundären Antikörper erfolgt.

Eine alternative Ausführungsform des erfindungsgemäßen Verfahrens betrifft einen nanopartikel-basierten Ansatz, der eine optische Kontrolle des Testosterongehaltes ohne weitere Hilfsmittel erlaubt. Hierzu werden mit Anti-Testosteron-Antikörper beschichtete Nanopartikel, insbesondere Gold-Nanopartikel, verwendet.

In einer speziellen Ausführungsform dieses Verfahrens werden die antikörperbeschichteten Nanopartikel mit einen multimeren Testosteronkonjugat kontaktiert und im Falle freier Bindungsplätze erfolgt eine Vernetzung von Nanopartikeln und Testosteronkonjugaten, welche ein detektierbares Signal, z.B. eine Farbänderung, erzeugt.

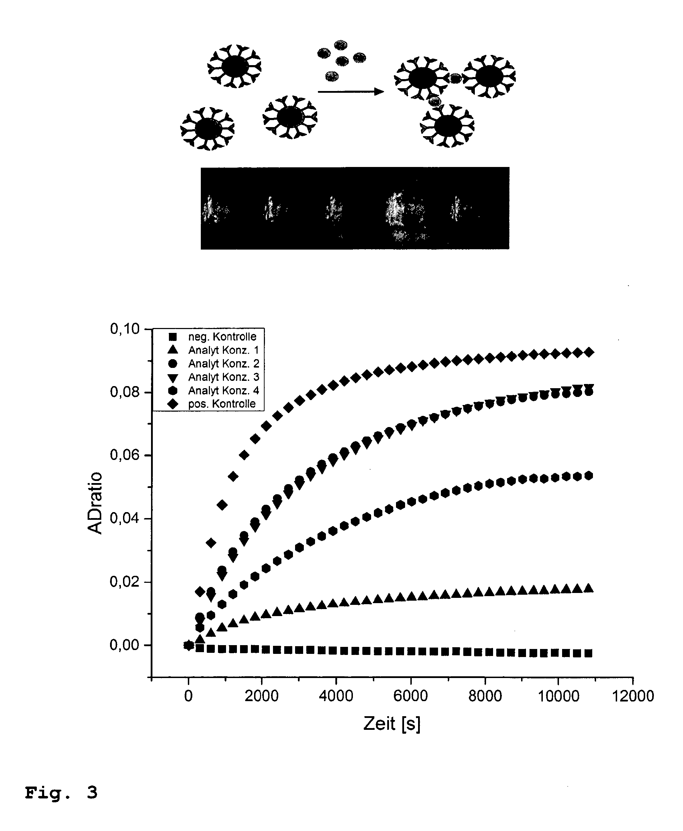

Hohe Testosteronkonzentrationen in der Probe bewirken hier wiederum die Absättigung der Paratope mit Testosteron. Eine niedrige Testosteronkonzentration bewirkt freie Bindungsplätze, welche zur Verfügung stehen, um in Anschluss mit dem multimeren Testosteronkonjugat eine Vernetzung durchzuführen, was zu einer Änderung des Absorptionsverhaltens führt und eine Farbänderung hervorruft. In Abb.3 ist das Prinzip schematisch dargestellt sowie die Farbänderung während der Aggregation.

Als Körperflüssigkeit kann grundsätzlich jede entnehmbare Körperflüssigkeit verwendet werden. Vorzugsweise handelt es sich dabei um Blut oder Speichel.

Speichel bietet den Vorteil einer einfachen Probennahme, die eine Zuordnung zum jeweiligen Tier problemlos ermöglicht. Der Nachweis in Blut stellt eine Möglichkeit dar, nach erfolgter Schlachtung eine Qualitätskontrolle im Schlachthof durchzuführen.

Eine bevorzugte Ausführungsform des erfindungsgemäßen Verfahrens ist dadurch gekennzeichnet, dass das detektierbare Signal mit Hilfe einer tragbaren Messeinrichtung erfasst und ausgewertet wird.

Typischerweise umfasst die tragbare Messeinrichtung dabei eine integrierte Kamera, eine Datenspeichereinheit sowie gegebenenfalls eine Datenübermittlungseinheit.

Es ist auch möglich, das erfindungsgemäße Verfahren so durchzuführen, dass neben Testosteron parallel noch die Anwesenheit von oder der Gehalt an weiteren Analyten festgestellt wird.

Ein weiterer Aspekt der Erfindung umfasst eine geeignete Vorrichtung zur Durchführung des erfindungsgemäßen Verfahrens.

Diese Vorrichtung umfasst mindestens
- eine Halterung für eine tragbare Messeinrichtung
- eine tragbare Messeinrichtung
- mindestens eine Haltevorrichtung für einen Reaktionsraum,
- mindestens einen Reaktionsraum.

Eine speziellere Ausführungsform dieser Vorrichtung ist dadurch gekennzeichnet, dass sich in der Halterung für eine tragbare Messeinrichtung eine Messeinrichtung befindet, welche eine Kamera, eine Datenspeichereinheit sowie gegebenenfalls eine Datenübermittlungseinheit umfasst, wobei der mindestens eine Reaktionsraum und die Aufnahmevorrichtung der Kamera räumlich so angeordnet sind, dass ein im Reaktionsraum erzeugtes optisches Signal direkt von der Kamera erfasst werden kann.

Ein weiterer Aspekt der Erfindung betrifft eine Komponente der obigen Vorrichtung, umfassend
- eine Halterung für eine tragbare Messeinrichtung
- mindestens eine Haltevorrichtung für einen Reaktionsraum,
- mindestens einen Reaktionsraum.

Eine speziellere Ausführungsform dieser Vorrichtung oder Komponente ist dadurch gekennzeichnet, dass der Reaktionsraum oberflächen-immobilisierte Analyten umfasst.

Typischerweise ist die erfindungsgemäße Vorrichtung oder Komponente dadurch gekennzeichnet, dass der Reaktionsraum über das Lambert-Beersche Gesetz definiert ist, d.h. einen definierten Lichtdurchlass benötigt bzw. aufweist. Der Reaktionsraum kann beispielsweise eine transparente Küvette sein.

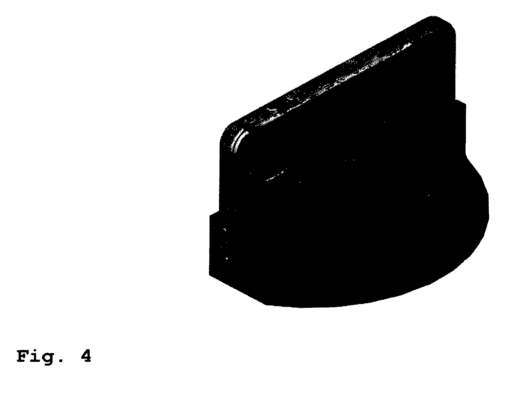

Fig. 4 zeigt beispielhaft eine solche Vorrichtung mit einer Halterung für eine tragbare Messeinrichtung (die z.B. ein handelsübliches Smartphone sein kann) mit zugehöriger Küvettenaufnahme. Der Assay wird in der Küvette durchgeführt und mit der integrierten Kamera aufgenommen und anschließend ausgewertet. Es kann außerdem die Identifikationsnummer des Tieres eingelesen werden, so dass eine sofortige Dokumentation der Ergebnisse erfolgen kann. Der Einsatz eines Managementsystems, welches eine Zuordnung von Schwein und Probe garantiert, ermöglicht einen erhöhten Durchsatz. Zusätzliche Küvettenaufnahmen können in diesem Fall am Halter integriert werden.

Die vorliegende Erfindung bietet, insbesondere in ihrer bevorzugten Ausführungsform zur Testosteronbestimmung bei Ebern, die folgenden Vorteile:
- Die Erfindung ermöglicht die Messung von Testosteron am "point of need". Der Vorteil besteht darin, dass keine Fehlschlachtungen vorkommen und finanzielle Verluste ausbleiben. Männliche Ferkel müssen nicht mehr kastriert werden, sofern eine Impfung erfolgt.
- Ein kontinuierliches Monitoring der Testosteronkonzentration ist möglich und damit auch der Einsatz in der "Jungebermast".
- Die Erfindung ermöglicht eine Kombination aus Messsystem und Managementsystem.
- Die Einfachheit der für die Erfindung genutzten Messapparatur erübrigt den Einsatz von speziellem Personal.
- Eine Messung mit dieser Erfindung ist innerhalb weniger Minuten möglich.
- Es besteht die Möglichkeit neben Testosteron auch andere Analyte bspw. für Krankheiten spezifische Moleküle zu detektieren. Dies ermöglicht ein Qualitätsmanagement.
- Der Einsatz von Nanopartikeln ermöglicht ein homogenes Assay-Prinzip ohne Spülschritte.

### Figurenbeschreibung

**Fig. 1** Schematische Darstellung eines erfindungsgemäßen kompetitiven Assays
**Fig. 2** Analyse des Testosteronspiegels von Schweinen
**Fig. 3** Vernetzung von Gold-Nanopartikeln
**Fig. 4** zeigt eine Vorrichtung zur Durchführung des erfindungsgemäßen Verfahrens mit einer Halterung für eine tragbare Messeinrichtung und einem Küvettenplatz.

### BEISPIEL 1

### Fluoreszenz-basierter Assay zum Testosteronnachweis

Eine Speichelprobe eines Test-Tiers wird mit einem anti-Testosteron-Antikörper gemischt. Eine niedrige Konzentration von Testosteron führt dazu, dass viele Paratop-Bindungsplätze unbesetzt bleiben und der anti-Testosteron-Antikörper für weitere Bindungsereignisse zur Verfügung steht. Im Anschluss daran erfolgt die Inkubation des anti-Testosteron-Antikörpers auf einem planaren Träger, der zuvor mit Testosteron beschichtet wurde. Im Falle, dass die Ausgangsprobe geringe Mengen Testosteron enthielt, findet eine hohe Anzahl an Bindungsereignissen statt. Mit einem fluoreszenzmarkierten sekundären Antikörper gegen den primären anti-Testosteron-Antikörper erfolgt der Nachweis der Bindungsereignisse an der beschichteten Oberfläche in Form eines Fluoreszenzsignals. Das allgemeine Assay-Prinzip ist in Abb.1 dargestellt. Für die Auslesung eines solchen Assays wurden bereits kostengünstige Systeme im IBMT entwickelt (Kozma et al., A novel handheld fluorescent microarray reader, in Biosensors and Bioelectronics (2013). Der hier verwendete Aufbau kann auch genutzt werden, um mehrere Analyte parallel zu bestimmen. Hierzu zählen bspw. Krankheitserreger.

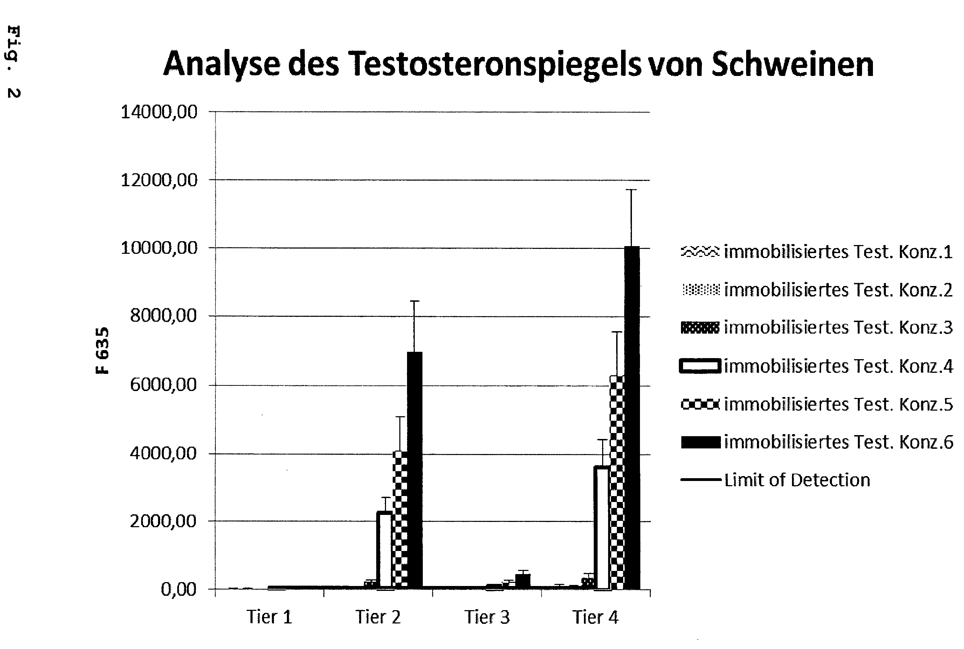

Es wurden Vollblutproben von Schweinen mit unterschiedlichem Testosterongehalt untersucht. Als Kontrolle diente das Vollblut einer Sau. Die Ergebnisse sind in Fig. 2 dargestellt. Tier 1 und Tier 3 entsprechen Ebern, welche hohe Testosteronkonzentrationen aufweisen und damit ein geringes Fluoreszenzsignal ergeben. Tier 2 entspricht einem Kastrat und Tier 4 einer Sau, welche geringe Testosteronkonzentrationen aufweisen und damit ein hohes Fluoreszenzsignal generieren.

### BEISPIEL 2

### Nanopartikel-basierter Assay zum Testosteronnachweis

Mit Anti-Testosteron-Antikörper beschichtete Gold-Nanopartikel werden hergestellt und mit einen multimeren Testosteronkonjugat kontaktiert (Arbeitsprotokoll nach Kumar, Aaron, Sokolov: Nature Protocols 2008, Vol.3, No. 2, S. 314).

Im Falle freier Bindungsplätze erfolgt eine Vernetzung von Nanopartikeln und Testosteronkonjugaten, was zu einer Änderung des Absorptionsverhaltens führt und die zuvor rot erscheinende kolloiddisperse Lösung blau färbt. In Fig.3 ist das Prinzip schematisch dargestellt sowie die Farbänderung während der Aggregation. Exemplarisch sind in dem rechtsstehenden Diagramm von Fig. 3 Vernetzungsversuche mit unterschiedlichen Konzentrationen eines multimeren Proteins gezeigt. Es wurde die ADRatio gegen die Zeit aufgetragen, wobei der Assay innerhalb von 10 min als abgeschlossen betrachtet werden kann.

## Patentansprüche

1. *In* vitro-Verfahren zur Bestimmung eines antikörperbasierten Analyten in Körperflüssigkeiten eines Säugers unter Verwendung eines Anti-Analyt-Antikörpers, welches die folgenden Schritte umfasst:
a) Kontaktieren einer Körperflüssigkeitsprobe des Säugers mit einem Anti-Analyt-Antikörper, wobei freier Analyt in der Probe an den Anti-Analyt-Antikörper gebunden wird und eine vollständige oder teilweise Absättigung der Analyt-Bindungsplätze des Antikörpers erfolgt,
b) Kontaktieren des nach Schritt a) erhaltenen Anti-Analyt-Antikörpers mit Referenz-Analyt und gegebenenfalls Bindung des Referenz-Analyten an noch vorhandene freie Bindungsplätze, wobei durch die Bindung ein detektierbares Signal erzeugt oder verändert wird,
c) Messen des erzeugten oder veränderten Signals, und
d) gegebenenfalls Vergleichen des gemessenen Signals mit Referenzwerten.

2. Verfahren nach Anspruch 1, **dadurch gekennzeichnet, dass** der Analyt Testosteron ist.

3. Verfahren nach Anspruch 1 oder 2, **dadurch gekennzeichnet, dass** der Referenz-Analyt auf einer Oberfläche immobilisiert oder als multimeres Analyt-Konjugat vorliegt.

4. Verfahren nach einem der Ansprüche 1 bis 3, **dadurch gekennzeichnet, dass** der Anti-Analyt-Antikörper in Schritt b) auch mit einem markierten sekundären Antikörper gegen den primären Anti-Analyt-Antikörper kontaktiert wird und die Bindung des markierten sekundären Antikörpers an das Konjugat von Anti-Analyt-Antikörper und Referenz-Analyt ein detektierbares Signal erzeugt oder verändert.

5. Verfahren nach einem der Ansprüche 1 bis 4, **dadurch gekennzeichnet, dass** das Signal ein optisches Signal ist.

6. Verfahren nach Anspruch 5, **dadurch gekennzeichnet, dass** der Referenz-Analyt auf einer Oberfläche immobilisiert ist und der Nachweis der Bindung des Anti-Analyt-Antikörpers an die Oberfläche mit einem markierten sekundären Antikörper erfolgt.

7. Verfahren nach einem der Ansprüche 1 bis 5, **dadurch gekennzeichnet, dass** mit Anti-Testosteron-Antikörper beschichtete Nanopartikel, insbesondere Gold-Nanopartikel, verwendet werden.

8. Verfahren nach Anspruch 7, **dadurch gekennzeichnet, dass** die antikörperbeschichteten Nanopartikel mit einen multimeren Testosteronkonjugat kontaktiert werden und im Falle freier Bindungsplätze eine Vernetzung von Nanopartikeln und Testosteronkonjugaten erfolgt, welche ein detektierbares Signal, z.B. eine Farbänderung, erzeugt.

9. Verfahren nach einem der Ansprüche 1 bis 8, **dadurch gekennzeichnet, dass** die Körperflüssigkeit Speichel oder Blut ist.

10. Verfahren nach einem der Ansprüche 1 bis 9, **dadurch gekennzeichnet, dass** das detektierbare Signal mit Hilfe einer tragbaren Messeinrichtung erfasst und ausgewertet wird.

11. Verfahren nach Anspruch 10, **dadurch gekennzeichnet, dass** die tragbare Messeinrichtung eine integrierte Kamera, eine Datenspeichereinheit sowie gegebenenfalls eine Datenübermittlungseinheit umfasst.

12. Verfahren nach einem der Ansprüche 1 bis 11, bei dem neben dem Testosteron parallel noch die Anwesenheit von oder der Gehalt an weiteren Analyten festgestellt wird.

13. Vorrichtung zur Durchführung des Verfahrens nach einem der Ansprüche 1 bis 12, umfassend mindestens
- eine Halterung für eine tragbare Messeinrichtung
- eine tragbare Messeinrichtung
- mindestens eine Haltevorrichtung für einen Reaktionsraum,
- mindestens einen Reaktionsraum.

14. Vorrichtung nach Anspruch 13, **dadurch gekennzeichnet, dass** sich in der Halterung für eine tragbare Messeinrichtung eine Messeinrichtung befindet, welche eine Kamera, eine Datenspeichereinheit sowie gegebenenfalls eine Datenübermittlungseinheit umfasst,
wobei der mindestens eine Reaktionsraum und die Aufnahmevorrichtung der Kamera räumlich so angeordnet sind, dass ein im Reaktionsraum erzeugtes optisches Signal direkt von der Kamera erfasst werden kann.

15. Komponente einer Vorrichtung nach Anspruch 13 oder 14, umfassend
- eine Halterung für eine tragbare Messeinrichtung
- mindestens eine Haltevorrichtung für einen Reaktionsraum,
- mindestens einen Reaktionsraum.

16. Vorrichtung nach Anspruch 13 oder 14 oder Komponente nach Anspruch 15, **dadurch gekennzeichnet, dass** der Reaktionsraum oberflächen-immobilisierte Analyten umfasst und/oder dass der Reaktionsraum über das Lambert-Beersche Gesetz definiert ist.
